# FASCICULE DE BREVET EUROPEEN

(11) **EP 1 348 422 B1**
(45) Date de publication et mention de la délivrance du brevet: **29.04.2009**
(21) Numéro de dépôt: 03291368.3
(22) Date de dépôt: 16.07.1998
(51) Int. Cl.: A61Q 5/06, A61K 8/41

(54) **Composition pour la teinture d'oxydation des fibres kératiniques comprenant du 2-chloro-6-méthyl-3-aminophénol et une base d'oxydation, et procédé de teinture**
2-Chlor-6-Methyl-3-Aminophenol und eine Oxidationsbase enthaltende Zusammensetzung zur oxidativen Färbung von Keratinfasern und Färbeverfahren
Oxidation dyeing composition for keratin fibers comprising 2-chloro-6-methyl-3-aminophenol and an oxidation base, and dyeing method

(30) Priorité: 01.09.1997 FR 9710854
(43) Date de publication de la demande: 01.10.2003
(62) Demande divisionnaire de: 98939696.5
(73) Titulaire: L'ORÉAL, 75008 Paris (FR)
(72) Inventeur: Audousset, Marie-Pascale, 92600 Asnières (FR)
(74) Mandataire: Fevrier, Murielle Françoise E.

(56) Documents cités:
- EP-A- 0 591 059
- EP-A- 0 728 465
- WO-A1-96/15765
- WO-A1-97/11674
- WO-A1-97/31886
- DE-A-
- DE-A- 3 016 008
- DE-A- 4 102 907
- DE-A- 4 205 329
- FR-A- 2 687 399
- W. UMBACH: "Kosmetik" 1995, GEORG THIEME VERLAG , STUTTGART-NEW YORK page 297

## Description

La présente invention a pour objet une composition pour la teinture d'oxydation des fibres kératiniques, en particulier des fibres kératiniques humaines telles que les cheveux, comprenant du 2-chloro 6-méthyl 3-aminophénol à titre de coupleur, en association avec au moins une base d'oxydation convenablement sélectionnée, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrimidine, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 016 008 des compositions pour la teinture d'oxydation des fibres kératiniques contenant à titre de coupleur du 2-chloro 6-méthyl 3-aminophénol ou du 2-méthyl 5-chloro 3-aminophénol, en association avec des bases d'oxydation classiquement utilisées pour la teinture d'oxydation, telles que par exemple certaines paraphénylènediamines, du para-aminophénol ou encore de la tétraaminopyrimidine. De telles compositions ne sont cependant pas entièrement satisfaisantes notamment du point de vue de la tenue des colorations obtenues vis à vis des diverses agressions que peuvent subir les cheveux et en particulier vis à vis des shampooings et des déformations permanentes.

Il a également été proposé, dans les demandes de brevet WO 96/15765 et WO 96/15766, des compositions pour la teinture d'oxydation des fibres kératiniques contenant l'association spécifique du 2-chloro 6-méthyl 3-aminophénol à titre de coupleur et de bases d'oxydation particulières comme la 2-β-hydroxyéthyl paraphénylènediamine ou certains para-aminophénols particuliers comme par exemple le 3-méthyl 4-aminophénol, le 2-allyl 4-aminophénol ou bien encore le 2-aminométhyl 4-aminophénol. De telles compositions ne sont cependant pas non plus entièrement satisfaisantes notamment du point de vue de la puissance des colorations obtenues. Il a aussi été proposé, dans la demande de brevet DE 42 05 329, une composition pour la coloration d'oxydation des cheveux comprenant une base d'oxydation choisie parmi des para-phénylènediamines de formule déterminée et un coupleur, par exemple le 2-chloro 6-méthyl 3-aminophénol, et dans la demande DE 41 02 907, une composition pour la coloration d'oxydation des cheveux comprenant une base d'oxydation choisie parmi les bis-(2,5-diaminophénoxy) oxoalcanes de formule déterminée et un coupleur, par exemple le 2-chloro 6-méthyl 3-aminophénol.

Or, la demanderesse vient maintenant de découvrir qu'il est possible d'obtenir de nouvelles teintures puissantes et particulièrement résistantes aux diverses agressions que peuvent subir les cheveux, en associant du 2-chloro 6-méthyl 3-aminophénol et au moins une base d'oxydation convenablement sélectionnée.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- du 2-chloro 6-méthyl 3-aminophénol et/ou au moins l'un de ses sels d'addition avec un acide, à titre de coupleur,
- et au moins une base d'oxydation choisie parmi :
   les orthoaminophénols ;
ladite composition étant exempte d'un coupleur additionnel qui serait choisi parmi la 4-hydroxy indoline, la 6-hydroxy indoline et leurs sels d'addition avec un acide.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment en ce qui concerne la résistance des colorations vis à vis des shampooings et des déformations permanentes.

Parmi les orthoaminophénols utilisables à titre de base d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Le 2-chloro 6-méthyl 3-aminophénol et/ou le ou ses sels d'addition avec un acide, représentent de préférence de 0,0001 à 5 % en poids environ du poids total de la composition tinctoriale, et encore plus préférentiellement de 0,005 à 3 % en poids environ de ce poids.

La ou les bases d'oxydation conformes à l'invention et/ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Les compositions tinctoriales conformes à l'invention peuvent contenir d'autres coupleurs différents du 2-chloro 6-méthyl 3-aminophénol et/ou des colorants directs notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention (bases d'oxydation) sont notamment ceux obtenus avec la soude, la potasse, l'ammoniaque ou les amines.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 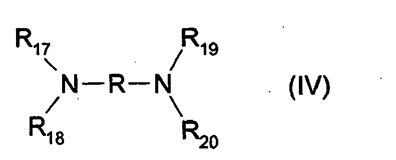 dans laquelle R est un reste propylène éventuellement substitué par un groupement hydroxyle ou un radical alkyle en C₁-C₄ ; R₁₇, R₁₈, R₁₉ et R₂₀, identiques ou différents, représentent un atome d'hydrogène, un radical alkyle en C₁-C₄ ou hydroxyalkyle en C₁-C₄.

La composition tinctoriale selon invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### Exemple de teinture

On a préparé la composition tinctoriale, conformes à l'invention, suivantes (teneurs en grammes) :

| **EXEMPLE** | 1 |
|---|---|
| 2-chloro 6-méthyl 3-aminophénol (coupleur) | 0,471 |
| Orthoaminophénol (base d'oxydation) | 0,327 |
| Support de teinture commun n°2 | (***) |
| Eau déminéralisée q.s.p. | 100 g |

| | |
|---|---|
| (***) support de teinture commun n°2: -Ethanol à 96 ° 18 g - Métabisulfite de sodium en solution aqueuse à 35 % 0,68 g - Sel pentasodique de l'acide diéthylènetriaminopentacétique 1,1 g - Ammoniaque à 20 % de NH₃ 10 g | |

Au moment de l'emploi, on a mélangé la composition tinctoriale ci-dessus avec une quantité égale en poids d'une composition oxydante constituée par une solution d'eau oxygénée à 20 volumes (6 % en poids).

La composition résultante a été appliquée pendant 30 minutes sur des mèches de cheveux gris naturels à 90 % de blancs. Les mèches de cheveux ont ensuite été rincées, lavées avec un shampooing standard puis séchées.

Les nuances obtenues figurent dans le tableau ci-dessous :

| **EXEMPLE** | **pH de TEINTURE** | **NUANCE OBTENUE** |
|---|---|---|
| 1 | 10 ± 0,2 | Doré cuivré |

## Revendications

1. Composition pour la teinture d'oxydation des fibres kératiniques humaines telles que les cheveux, **caractérisée par le fait qu'**elle comprend, dans un milieu approprié pour la teinture :
- du 2-chloro 6-méthyl 3-aminophénol et/ou au moins l'un de ses sels d'addition avec un acide, à titre de coupleur,
- et au moins une base d'oxydation choisie parmi les orthoaminophénols ;
ladite composition étant exempte d'un coupleur additionnel qui serait choisi parmi la 4-hydroxy indoline, la 6-hydroxy indoline et leurs sels d'addition avec un acide.

2. Composition selon la revendication 1, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

3. Composition selon la revendication 1 ou 2, **caractérisée par le fait que** le 2-chloro 6-méthyl 3-aminophénol et/ou le ou ses sels d'addition avec un acide représente de 0,0001 à 5% en poids du poids total de la composition tinctoriale.

4. Composition selon la revendication 3, **caractérisée par le fait que** le 2-chloro 6-méthyl 3-aminophénol et/ou le ou ses sels d'addition avec un acide représente de 0,005 à 3% en poids du poids total de la composition tinctoriale.

5. Composition selon l'une quelconque des revendications précédentes, **caractérisée par le fait que** la ou les bases d'oxydation représentent de 0,0005 à 12% en poids du poids total de la composition tinctoriale.

6. Composition selon la revendication 5, **caractérisée par le fait que** la ou les bases d'oxydation représentent de 0,005 à 6% en poids du poids total de la composition tinctoriale.

7. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates et que les sels d'addition avec une base sont ceux obtenus avec la soude, la potasse, l'ammoniaque ou les amines.

8. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

9. Composition selon l'une quelconque des revendications précédentes, **caractérisée par le fait qu'**elle présente un pH compris entre 3 et12.

10. Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

11. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 10, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

12. Procédé selon la revendication 11, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates, les peracides.

13. Dispositif à plusieurs compartiments, ou "kit" de teinture à plusieurs compartiments, dont un premier compartiment renferme une composition tinctoriale telle que définie à l'une quelconque des revendications 1 à 10 et un second compartiment renferme une composition oxydante.

## Claims

1. Composition for the oxidation dyeing of human keratinous fibres, such as hair, **characterized in that** it comprises, in a medium appropriate for dyeing:
- 2-chloro-6-methyl-3-aminophenol and/or at least one of its addition salts with an acid, as coupler, and
- at least one oxidation base chosen from ortho-aminophenols;
the said composition being devoid of an additional coupler which would be chosen from 4-hydroxyindoline, 6-hydroxyindoline and their addition salts with an acid.

2. Composition according to Claim 1, **characterized in that** the ortho-aminophenols are chosen from 2-aminophenol, 2-amino-5-methylphenol, 2-amino-6-methylphenol, 5-acetamido-2-aminophenol, and their addition salts with an acid.

3. Composition according to Claim 1 or 2,
**characterized in that** 2-chloro-6-methyl-3-aminophenol and/or its addition salt or salts with an acid represent from 0.0001 to 5% by weight of the total weight of the dyeing composition.

4. Composition according to Claim 3, **characterized in that** 2-chloro-6-methyl-3-aminophenol and/or its addition salt or salts with an acid represent from 0.005 to 3% by weight of the total weight of the dyeing composition.

5. Composition according to any one of the preceding claims, **characterized in that** the oxidation base or bases represent from 0.0005 to 12% by weight of the total weight of the dyeing composition.

6. Composition according to Claim 5, **characterized in that** the oxidation base or bases represent from 0.005 to 6% by weight of the total weight of the dyeing composition.

7. Composition according to any one of the preceding claims, **characterized in that** the addition salts with an acid are chosen from hydrochlorides, hydrobromides, sulphates and tartrates, lactates and acetates and **in that** the addition salts with a base are those obtained with sodium hydroxide, potassium hydroxide, ammonia or amines.

8. Composition according to any one of the preceding claims, **characterized in that** the medium appropriate for dyeing (or vehicle) is composed of water or of a mixture of water and at least one organic solvent chosen from lower C₁-C₄ alkanols, glycerol, glycols and glycol ethers, aromatic alcohols, analogous products and their mixtures.

9. Composition according to any one of the preceding claims, **characterized in that** it exhibits a pH of between 3 and 12.

10. Composition according to any one of the preceding claims, **characterized in that** it is provided in the form of liquids, creams or gels, or in any other form appropriate for carrying out dyeing of keratinous fibres and in particular of human hair.

11. Process for dyeing keratinous fibres and in particular human keratinous fibres, such as hair, **characterized in that** at least one dyeing composition as defined in any one of Claims 1 to 10 is applied to these fibres and **in that** the colour is developed at acidic, neutral or alkaline pH using an oxidizing agent which is added only at the time of use to the dyeing composition or which is present in an oxidizing composition applied simultaneously or sequentially in a separate fashion.

12. Process according to Claim 11, **characterized in that** the oxidizing agent present in the oxidizing composition is chosen from hydrogen peroxide, urea hydrogen peroxide, alkali metal bromates, persalts, such as perborates and persulphates, or peracids.

13. Dyeing multi-compartment device or kit with several compartments, a first compartment of which includes a dyeing composition as defined in any one of Claims 1 to 10 and a second compartment of which includes an oxidizing composition.

## Patentansprüche

1. Zusammensetzung zur Oxidationsfärbung von menschlichen Keratinfasern wie der Haare, **dadurch gekennzeichnet, dass** sie in einem zum Färben geeigneten Medium enthält:
- 2-Chlor-6-methyl-3-aminophenol und/oder mindestens eines seiner Additionssalze mit einer Säure als Kuppler und
- mindestens eine Oxidationsbase, die unter den o-Aminophenolen ausgewählt ist,
wobei die Zusammensetzung keinen unter 4-Hydroxyindolin und 6-Hydroxyindolin sowie ihren Additionssalzen mit einer Säure ausgewählten zusätzlichen Kuppler enthält.

2. Zusammensetzung nach Anspruch 1, **dadurch gekennzeichnet, dass** die o-Aminophenole ausgewählt sind unter 2-Aminophenol, 2-Amino-5-methylphenol, 2-Amino-6-methylphenol und 5-Acetamido-2-aminophenol sowie ihren Additionssalzen mit einer Säure.

3. Zusammensetzung nach Anspruch 1 oder 2, **dadurch gekennzeichnet, dass** das 2-Chlor-6-methyl-3-aminophenol und/oder das Additionssalz oder seine Additionssalze mit einer Säure 0,0001 bis 5 Gew.-% des Gesamtgewicht der Färbezusammensetzung ausmachen.

4. Zusammensetzung nach Anspruch 3, **dadurch gekennzeichnet, dass** das 2-Chlor-6-methyl-3-aminophenol und/oder das oder seine Additionssalze mit einer Säure 0,005 bis 3 Gew.-% des Gesamtgewichts der Färbezusammensetzung ausmachen.

5. Zusammensetzung nach einem der vorhergehenden Ansprüche, **dadurch gekennzeichnet, dass** die Oxidationsbase oder die Oxidationsbasen 0,0005 bis 12 Gew.-% des Gesamtgewichts der Färbezusammensetzung ausmachen.

6. Zusammensetzung nach Anspruch 5, **dadurch gekennzeichnet, dass** die Oxidationsbase oder die Oxidationsbasen 0,005 bis 6 Gew.-% des Gesamtgewichts der Färbezusammensetzung ausmachen.

7. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind und dass die Additionssalze mit einer Base mit Natriumhydroxid, Kaliumhydroxid, Ammoniak oder Aminen erhalten sind.

8. Zusammensetzung nach einem der vorhergehenden Ansprüche, **dadurch gekennzeichnet, dass** das zum Färben geeignete Medium (oder der Träger) aus Wasser oder einem Gemisch von Wasser mit mindestens einem organischen Lösungsmittel besteht, das ausgewählt ist unter niederen C₁-C₄-Alkanolen, Glycerin, Glycolen und Glycolethern, aromatischen Alkoholen, analogen Produkten sowie ihren Gemischen.

9. Zusammensetzung nach einem der vorhergehenden Ansprüche, **dadurch gekennzeichnet, dass** sie einen pH-Wert von 3 bis 12 aufweist.

10. Zusammensetzung nach einem der vorhergehenden Ansprüche, **dadurch gekennzeichnet, dass** sie in Form von Flüssigkeiten, Cremen oder Gelen oder in einer beliebigen anderen Form vorliegt, die zur Durchführung einer Färbung von Keratinfasern und insbesondere der menschlichen Haare geeignet ist.

11. Verfahren zum Färben von Keratinfasern und insbesondere von menschlichen Keratinfasern wie der Haare, **dadurch gekennzeichnet, dass** auf diese Fasern mindestens eine Färbezusammensetzung, wie sie in einem der Ansprüche 1 bis 10 definiert ist, aufgebracht wird und die Färbung bei einem sauren, neutralen oder alkalischen pH-Wert mit einem Oxidationsmittel entwickelt wird, das unmittelbar bei der Anwendung zu der Färbezusammensetzung zugegeben wird oder das in einer oxidierenden Zusammensetzung vorliegt, die gleichzeitig oder anschließend getrennt aufgebracht wird.

12. Verfahren nach Anspruch 11, **dadurch gekennzeichnet, dass** das in der oxidierenden Zusammensetzung vorliegende Oxidationsmittel unter Wasserstoffperoxid, Harnstoffperoxid, Alkalimetallbromaten, Persalzen wie Perboraten und Persulfaten und Persäuren ausgewählt ist.

13. Vorrichtung mit mehreren Compartments oder "Kit" zum Färben mit mehreren Compartments, wovon ein erstes Compartment eine Färbezusammensetzung, wie sie in einem de r Ansprüche 1 bis 10 definiert ist, und ein zweites Compartment eine oxidierende Zusammensetzung enthält.
